Europäisches Patentamt

European Patent Office

Office européen des brevets

(19)

(11) Numéro de publication : **0 471 054 B1**

(12) ## FASCICULE DE BREVET EUROPEEN

(45) Date de publication du fascicule du brevet :
**04.05.94 Bulletin 94/18**

(51) Int. Cl.⁵ : **A61K 7/032,** A61K 7/48, A61K 7/06

(21) Numéro de dépôt : **91905453.6**

(22) Date de dépôt : **21.02.91**

(86) Numéro de dépôt international :
**PCT/FR91/00142**

(87) Numéro de publication internationale :
**WO 91/12793 05.09.91 Gazette 91/21**

(54) COMPOSITION RESISTANTE A l'EAU POUR LE REVETEMENT DES CILS, ET SON PROCEDE DE PREPARATION.

(30) Priorité : **01.03.90 FR 9002578**

(43) Date de publication de la demande :
**19.02.92 Bulletin 92/08**

(45) Mention de la délivrance du brevet :
**04.05.94 Bulletin 94/18**

(84) Etats contractants désignés :
**AT BE CH DE DK ES FR GB IT LI NL SE**

(56) Documents cités :
**FR-A- 2 528 699
GB-A- 2 124 081
GB-A- 2 167 301
GB-A- 2 216 797
WPI, File Supplier, AN = 83-829918, Derwent
Publications Ltd., (Londres, GB), &
JP-A-58180412 (Kobayashi Kosei K.K.), 21
octobre 1983
Hawley's Condensed Chemical Dictionary,
11ème édition, p. 973**

(73) Titulaire : **L'OREAL
14, Rue Royale
F-75008 Paris (FR)**

(72) Inventeur : **ARRAUDEAU, Jean-Pierre
308, rue Lecourbe
F-75015 Paris (FR)**
Inventeur : **PATRAUD, Jeanne
Tour Palerme, 1216, boulevard Masséna
F-75013 Paris (FR)**
Inventeur : **PIOT, Bertrand
13, rue du Transvaal
92250 La Garenne Colombes (FR)**

(74) Mandataire : **Peuscet, Jacques
Cabinet Peuscet 68, rue d'Hauteville
F-75010 Paris (FR)**

EP 0 471 054 B1

Jouve, 18, rue Saint-Denis, 75001 PARIS

## Description

La présente invention porte sur une composition cosmétique résistante à l'eau, pour le revêtement des cils. Une telle composition, lorsqu'elle est destinée au maquillage des cils, contient généralement des pigments et est alors appelée "mascara" ; dans le cas où elle ne comporte pas de pigments, elle constitue simplement une base de maquillage des cils ou une base traitante pour les cils. La présente invention porte également sur un procédé de préparation de cette nouvelle composition.

Les mascaras résistants à l'eau, qui sont actuellement sur le marché, sont des produits anhydres à base de solvants organiques non-aqueux. L'inconvénient majeur de ces mascaras est leur médiocre propriété d'allongement, inférieure, dans tous les cas, à celle des mascaras non résistants à l'eau. Il existe aussi des mascaras résistants à l'eau se présentant sous la forme d'émulsions eau-dans-l'huile ; cependant, leur résistance à l'eau n'est pas satisfaisante.

Il est connu par GB-A 2 167 301, FR-A 2 528 699 de préparer des mascaras constitués par une émulsion de cires dans l'eau, qui ne sont pas résistants à l'eau, contenant un polymère filmogène hydrosoluble et un agent émulsionnant. Il est également connu par GB-A 2 216 797 de préparer un mascara résistant à l'eau, contenant une solution aqueuse de polymère filmogène hydrosoluble et un agent émulsionnant. Ce document ne permettait, par conséquent, pas de prévoir qu'en ajoutant un polymère filmogène on pourrait obtenir un mascara résistant à l'eau en l'absence d'agent émulsionnant.

La société déposante a constaté, d'une façon tout à fait surprenante, que, lorsqu'on introduit, dans une composition de mascara résistante à l'eau anhydre typique, une solution aqueuse d'au moins un polymère filmogène hydrosoluble, il est possible d'augmenter de façon substantielle la résistance à l'eau du mascara, d'obtenir simultanément une composition qui, bien que contenant de l'eau dans un milieu parfaitement hydrophobe, soit stable sans la présence d'un agent émulsionnant, et également d'obtenir un mascara ayant des propriétés cosmétiques supérieures à celles des mascaras résistants à l'eau connus à ce jour, notamment du point de vue de la rapidité du maquillage, de la facilité d'application, de l'allongement et du recourbement des cils. Il faut noter que l'addition du polymère filmogène en l'absence d'eau ne permet pas d'améliorer les qualités du mascara. La présence d'eau dans le polymère filmogène est donc nécessaire.

L'introduction d'une solution aqueuse de substances hydrosolubles, ou d'eau, dans des produits cosmétiques normalement anhydres a déjà été décrite, en particulier dans la demande de brevet japonais n° 61/83110, mais il s'agit, dans ce cas, d'un produit cosmétique à usage cutané, en particulier d'un rouge à lèvres, dont le but est l'apport de substances hydratantes et le dépôt d'un film gras et humide sur les lèvres ; selon ce document, on disperse de l'eau ou une solution aqueuse d'une substance hydrosoluble, dans un rouge à lèvres ou une base de rouge à lèvres, en présence d'un ou de plusieurs agents dispersants choisis parmi le cholestérol, les phytostérols, les phospholipides et les saponines. La présence d'agents dispersants, tels que les stérols, est indispensable à la réalisation et à la stabilité de ces produits, car lesdits dispersants permettent d'incorporer la phase aqueuse dans la base anhydre.

Il a été vérifié que la composition cosmétique de maquillage pour les cils selon l'invention est tout à fait réalisable en l'absence de stérols, lesquels peuvent être néanmoins présents dans la formule à de très faibles concentrations, par l'intermédiaire de cires.

La présente invention a donc pour objet le produit industriel nouveau que constitue une composition résistante à l'eau, pour le revêtement des cils, contenant au moins une cire, au moins un agent de consistance, et au moins un solvant organique volatil, caractérisée par le fait qu'elle contient, en outre, de 1 à 35 % par rapport au poids total de la composition d'une solution aqueuse d'au moins un polymère filmogène hydrosoluble, la concentration en polymère(s) filmogène(s) hydrosoluble(s) dans la solution étant comprise entre 0,1 et 55 % en poids de matière active et qu'elle ne contient pas d'agent émulsionnant.

Le (ou les) polymère(s) filmogène(s) est (ou sont) choisi(s), notamment, dans le groupe formé par:
- les dérivés de kératine, tels que les hydrolysats de kératine et les kératines sulfoniques ;
- les dérivés de chitine ou de chitosane anioniques, cationiques, amphotères ou non ioniques;
- les dérivés de cellulose, tels que l'hydroxy-éthylcellulose, l'hydroxypropylcellulose, la méthyl cellulose, l'éthylhydroxyéthylcellulose, la carboxyméthylcellulose, ainsi que les dérivés quaternisés de la cellulose ;
- les polymères acryliques, tels que les polyacrylates et les polyméthacrylates, ainsi que les copolymères acryliques ;
- les polyvinylpyrrolidones et les copolymères vinyliques, tels que le copolymère de l'éther méthylvinylique et de l'anhydride malique, ou le copolymère de l'acétate de vinyle et de l'acide crotonique;
- les polymères naturels, tels que :
  . les gommes arabiques, la gomme de guar, les dérivés du xanthane et la gomme de karaya ;
  . les alginates et les carraghénates ;

2

. les glycoaminoglycanes, l'acide hyaluronique et ses dérivés ;
- les polymères de l'éthylène, tels que les polyéthylèneglycols; et
- les silicones oxyéthylénées.

La (ou les) cire(s) est (ou sont) choisie(s) notamment parmi les cires animales, végétales, minérales, synthétiques et les fractions diverses de cires naturelles, toutes ces cires ayant, en règle générale, un point de fusion compris entre 60 et 110°C, et une pénétration à l'aiguille, à 25°C, comprise entre environ 3 et 40, telle que mesurée selon la norme américaine ASTM D5 ou selon la norme française NFT 004. Le principe de la mesure de la pénétration d'une aiguille selon ces deux normes consiste à mesurer la profondeur, exprimée en dixièmes de millimètre, à laquelle pénètre une aiguille normalisée (pesant 2,5 g, placée dans un porte-aiguille pesant 47,5 g, soit au total, 50 g), placée sur la cire pendant 5 secondes.

Parmi les cires animales que l'on peut utiliser, on peut citer entre autres les cires d'abeille, les cires de lanoline et les cires d'insecte de Chine. Parmi les cires végétales, on peut citer, entre autres, les cires de Carnauba, de Candelilla, d'Ouricurry, les cires de fibres de liège, les cires de canne à sucre et les cires du Japon. Parmi les cires minérales, on peut citer, en particulier, les paraffines, les cires microcristallines, les cires de lignite (Montan wachs) et les ozokérites. Parmi les cires synthétiques, on peut citer, en particulier, les cires de polyéthylène, les cires obtenues par la synthèse de Fischer et Tropsch, et les polymères cireux ainsi que leurs esters. Toutes ces cires sont bien connues de l'homme du métier.

De préférence, la (ou les) cire(s) utilisée(s) selon l'invention est (ou sont) solide(s) et rigide(s) à une température inférieure à 50°C. De plus, la concentration en cire(s), par rapport au poids total de la composition, est comprise notamment entre environ 2 et 40 % en poids.

Le (ou les) agent(s) de consistance est (ou sont) choisi(s) notamment dans le groupe formé par les argiles modifiées organiquement, telles que les montmorillonites et les dérivés d'hectorite, par exemple, la bentonite. La concentration en agent(s) de consistance par rapport au poids total de la composition est comprise notamment entre environ 5 et 15 % en poids.

Le (ou les) solvant(s) organique(s) volatil(s) est (ou sont) choisi(s) notamment dans le groupe formé par l'isoparaffine, l'essence de térébenthine, l'alcool isopropylique, l'alcool éthylique, le white spirit et les dérivés de silicone volatils ; la concentration en solvant(s) organique(s) volatil(s) par rapport au poids total de la composition est comprise notamment entre environ 35 et 50 % en poids.

Par ailleurs, la composition selon l'invention peut également contenir jusqu'à 10 % en poids, par rapport au poids total de la composition, d'au moins une charge. Les charges sont essentiellement destinées à augmenter les caractéristiques de couvrance du produit et sont notamment les poudres habituellement utilisées dans les produits cosmétiques, telles que le talc, l'amidon, le kaolin et les polyamides.

La composition selon l'invention peut également contenir au moins un pigment, dans une proportion pouvant aller jusqu'à 20 % en poids par rapport au poids total de la composition, suivant la coloration et l'intensité de la coloration que l'on cherche à obtenir. On peut cependant, comme indiqué ci-dessus, envisager de réaliser une composition sans pigments, laquelle constitue alors une base de maquillage des cils ou une base traitante résistante à l'eau, pour les cils.

Les pigments utilisables sont choisis notamment parmi les pigments minéraux, les pigments organiques, les pigments nacrés et les pigments enrobés.

Parmi les pigments minéraux, on peut citer, à titre d'exemples :
- le dioxyde de titane (rutile ou anatase), éventuellement traité en surface et codifié dans le Color Index sous la référence CI 77891 ;
- les oxydes de fer noir, jaune, rouge et brun, codifiés sous les références CI 77499, 77492, 77491 ;
- le violet de manganèse (CI 77742) ;
- le bleu outremer (CI 77007) ;
- l'oxyde de chrome (CI 77288) ;
- l'hydrate de chrome (CI 77289) ; et
- le bleu ferrique (CI 77510).

Parmi les pigments organiques, on peut citer, en particulier les pigments certifiés aux Etats-Unis d'Amérique par la FOOD & DRUG ADMINISTRATION sous les dénominations :
- D & C red n° 19 (CI 45170) ;
- D & C red n° 9 (CI 15585) ;
- D & C red n° 30 (CI 73360) ;
- D & C red n° 3 (CI 45430) ;
- D & C red n° 21 (CI 45380) ;
- D & C red n° 27 (CI 45410) ;
- D & C red n° 13 (CI 15630) ;
- D & C red n° 7 (CI 15850 - 1) ;

EP 0 471 054 B1

- D & C red n° 6 (CI 15850 - 2) ;
- D & C red n° 36 (CI 12085) ;
- D & C orange n° 10 (CI 45425) ;
- D & C orange n° 4 (CI 15510) ;
- D & C orange n° 5 (CI 45370) ;
- D & C yellow n° 6 (CI 15985) ;
- D & C yellow n° 5 (CI 19140) ;

ainsi que :
- le noir de carbone (CI 77266) ; et
- les laques à base de carmin de cochenille (CI 75470).

Les pigments nacrés peuvent etre choisis notamment parmi :
- les pigments nacrés blancs, tels que le mica recouvert d'oxyde de titane, l'oxychlorure de bismuth ; et
- les pigments nacrés colorés, tels que le mica titane avec des oxydes de fer, le mica titane avec du bleu ferrique, ou de l'oxyde de chrome, le mica titane avec un pigment organique du type précité, ainsi que ceux à base d'oxychlorure de bismuth ;
- les pigments enrobés tels que ceux obtenus à partir des pigments listés ci-dessus et dont la surface a été traitée par diverses substances comme, par exemple, des acides aminés, des silicones, des sels métalliques ou du collagène.

Les compositions selon l'invention peuvent également contenir, en plus des composants mentionnés précédemment, des ingrédients utilisés de façon classique dans les compositions de maquillage pour les cils, et choisis notamment parmi les adoucissants, les conservateurs, les séquestrants, les parfums, les épaississants, les huiles, les silicones, les agents de cohésion, les polymères non-filmogènes, les agents alcalinisants ou acidifiants, et les agents reconnus pour leur action bénéfique sur les cils, tels que les vitamines ou les acides aminés.

La présente invention a également pour objet un procédé de préparation d'une composition résistante à l'eau pour le revêtement des cils, telle qu'elle a été définie ci-dessus, ce procédé étant caractérisé par le fait que :
- dans une première étape, on mélange les composants de la phase grasse et les éventuels additifs liposolubles ;
- dans une seconde étape, on ajoute au mélange ainsi obtenu, les charges et/ou pigments éventuels, puis le (ou les) solvant(s) organique(s) volatil(s);

et
- dans une troisième étape, on disperse dans le mélange résultant, la phase aqueuse contenant le (ou les) polymère(s) filmogène(s) hydrosoluble(s) et les éventuels additifs et/ou ingrédients actifs hydrosolubles.

Pour mieux faire comprendre l'objet de l'invention, on va en décrire maintenant, à titre d'exemples purement illustratifs, plusieurs modes de mise en oeuvre. Les exemples 1 à 9 sont des exemples de formulation de différents mascaras qui sont préparés conformément au mode opératoire général défini ci-dessus. Ces mascaras, bien que ne contenant pas d'émulsionnant, sont stables; ils ont été appliqués sur des cils par des utilisatrices et ils ont tous donné satisfaction. Les exemples 10 à 12 décrivent des tests comparatifs et les résultats entre des mascaras de l'invention et un mascara classique résistant à l'eau, la différence étant la présence, dans les premiers, d'une solution aqueuse de polymère filmogène hydrosoluble.

Exemple 1 :

On prépare un mascara résistant à l'eau formulé comme suit :

```
Cire de paraffine ........................... 12  g
Alcool de lanoline .......................... 15  g
Amidon ....................................... 2  g
```

```
Oxyde de fer ...................................  5   g
Isoparaffine .................................. 45   g
Montmorillonite ...............................  8   g
Panthénol .....................................  3   g
Pyrrolidone carboxylate de chitosonium, vendu
sous la dénomination "KYTAMER PC" par la
société "AMERCHOL" ............................  3   g
Eau ...........................................  7   g
Conservateurs ................................. qs
```

Exemple 2 :

On prépare un mascara résistant à l'eau formulé comme suit :

```
Cire de Carnauba ............................. 12   g
Alcool de lanoline ........................... 15   g
Amidon .......................................  2   g
Oxyde de fer .................................  5   g
Isoparaffine ................................. 45   g
Montmorillonite ..............................  8   g
Hydrolysat de kératine vendu sous la
dénomination "KERASOL" par la société
"CRODA CHEMICALS"............................  2,5 g
Eau ......................................... 10,5 g
Conservateurs ............................... qs
```

Exemple 3 :

On prépare un mascara résistant à l'eau formulé comme suit :

```
Cire d'abeille naturelle .................... 12   g
Paraffine ................................... 15   g
Amidon ......................................  2   g
Oxyde de fer ................................  5   g
Isoparaffine ................................ 45   g
Montmorillonite .............................  8   g
Cystéine .................................... 0,5 g
Hydroxyproline .............................. 0,5 g
Copolymère méthochlorure de vinylimidazolinium/
pyrrolidone vinylique (rapport en poids : 30/70),
```

vendu sous la dénomination "LUVIQUAT FC 370" par
la société "BASF" ............................... 4 g

Eau .............................................. 8 g

Conservateurs .................................... qs

Exemple 4 :

On prépare un mascara résistant à l'eau formulé comme suit :

Paraffine ................................. 12 g
Alcool de lanoline ........................ 15 g
Talc ...................................... 2 g
Oxyde de fer .............................. 5 g
Isoparaffine .............................. 45 g
Montmorillonite ........................... 8 g
Copolymère acrylamide/chlorure de diméthyl
diallylammonium en solution dans l'eau, à
7 % de matières sèches, vendu sous la
dénomination "MERQUAT 550" par la société
"MERCK" ................................... 2 g
Eau ....................................... 11 g
Conservateurs ............................. qs

Exemple 5 :

On prépare un mascara résistant à l'eau formulé comme suit :

Cire d'abeille modifiée ................... 10 g
Paraffine ................................. 10 g
Amidon .................................... 2 g
Oxyde de fer .............................. 7 g
Isoparaffine .............................. 50 g
Montmorillonite ........................... 8 g
Hydroxyéthylcellulose/chlorure de diallyl
diméthyl ammonium, vendu sous la dénomination
"CELQUAT L200" par la société
"NATIONAL STARCH" ......................... 1,5 g
Eau ....................................... 11,5 g
Conservateurs ............................. qs

Exemple 6 :

On prépare un mascara résistant à l'eau formulé comme suit :

Cire d'abeille naturelle ........................ 10    g

Paraffine .......................................  6    g

Ozokérite ......................................  6    g

Talc ...........................................  2    g

Oxyde de fer ...................................  5    g

Montmorillonite ................................  8    g

Isoparaffine ................................... 50    g

Copolymère de pyrrolidone vinylique/acétate
de vinyle, vendu sous la dénomination
"PVP/VA W-735" par la société "GAF CORP." .....  3    g

Eau ............................................ 10    g

Conservateurs ................................... qs

Exemple 7 :

On prépare un mascara résistant à l'eau formulé comme suit :

| Cire de paraffine | 10 g |
| Alcool de lanoline | 13 g |
| Amidon | 2 g |
| Oxyde de fer | 5 g |
| Isoparaffine | 41,5 g |
| Montmorillonite | 8 g |
| Panthénol | 5 g |
| Gomme arabique | 8,5 g |
| Eau | 7 g |
| Conservateurs | qs |

Exemple 8 :

On prépare un mascara résistant à l'eau formulé comme suit :

Cire de paraffine ............................. 12    g

Acide stéarique ............................... 17    g

Amidon ........................................  1    g

Oxyde de fer ..................................  5    g

Isoparaffine .................................. 44,5  g

Montmorillonite ............................... 10    g

Pyrrolidone carboxylate de chitosonium, vendu sous
la dénomination "KYTAMER PC" par la société
"AMERCHOL" ....................................  1,5  g

Eau ...........................................  9    g

Conservateurs .................................. qs

Exemple 9 :

On prépare un mascara résistant à l'eau formulé comme suit :

7

```
Cire de paraffine ............................ 12   g
Acide oléique ................................ 12   g
Huile végétale ...............................  6   g
Amidon .......................................  1   g
Oxyde de fer .................................  5   g
Isoparaffine ................................. 41   g
Montmorillonite .............................. 10   g
Hydroxyproline ............................... 0,5 g
Cystéine ..................................... 0,5 g
Hydroxypropylméthylcellulose vendue sous la
dénomination "METHOCEL E" par la société
"DOW CHEMICAL" ...............................  2   g
Eau .......................................... 10   g
Conservateurs ................................ qs
```

EXEMPLE 10

On prépare un mascara résistant à l'eau formulé comme suit :

```
Cire d'abeilles naturelle .................... 12    g
Paraffine .................................... 10    g
Cire de Carnauba .............................  7    g
Amidon .......................................  2    g
Oxyde de fer .................................  5    g
Isoparaffine ................................. 47,8  g
Montmorillonite .............................. 7,5   g
Glutamate de chitosane vendu sous
la dénomination "SEA CURE 110"

par la société "PROTAN" ...................... 0,7 g
Eau ..........................................  8    g
Conservateurs ................................ qs
```

Exemple 11 : (Comparatif)

On a demandé à 86 utilisatrices de tester successivement les deux mascaras suivants, l'applicateur étant identique :

1) Mascara A : mascara classique résistant à l'eau, ayant la formulation suivante :

| | |
|---|---|
| Cire de Carnauba | 13,8 g |
| Alcool de lanoline | 17,2 g |
| Amidon | 2,3 g |
| Oxyde de fer | 5,7 g |
| Isoparaffine | 51,8 g |
| Montmorillonite | 9,2 g |

2) Mascara B : mascara selon l'invention correspondant à la formulation du mascara "A" où l'on a ajouté 10 % en poids, d'une solution aqueuse (à 13 % en poids de matières sèches) d'un hydrolysat de kératine

8

vendu sous la dénomination "KERASOL" par la société "CRODA CHEMICALS".

On leur a ensuite demandé de donner leur avis sur différents paramètres, et de noter sur 10 ces deux mascaras, critère par critère, et globalement. Les avis exprimés en pourcentage et les notes ainsi données figurent dans le Tableau ci-après :

| Paramètres | | Mascara A % des avis exprimés | Mascara B % des avis exprimés |
|---|---|---|---|
| Facilité d'application | Facile | 51 | 71 |
| | Assez facile | 19 | 14 |
| | Plutôt difficile | 17 | 14 |
| | Difficile | 13 | 1 |
| | Note moyenne/10 | 6,36 | 7,40 |
| Charge | Bonne | 44 | 61 |
| | Trop importante | 20 | 22 |
| | Insuffisante | 34 | 15 |
| | Irrégulière | 2 | 2 |
| | Note moyenne/10 | 6,03 | 7,06 |
| Allongement | Bon | 46 | 64 |
| | Assez bon | 21 | 20 |
| | Insuffisant | 22 | 12 |
| | "Inexistant" | 11 | 4 |
| | Note moyenne/10 | 6,42 | 7,45 |
| Recourbement | Bon | 44 | 63 |
| | Moyen | 36 | 28 |
| | Insuffisant | 20 | 9 |
| | Note moyenne/10 | 6,30 | 7,34 |

Ce tableau montre que le mascara B est au total nettement préféré au mascara A.

Exemple 12 : (Comparatif)

On compare la rémanence à l'eau de différents mascaras.

Le test effectué est basé sur le fait que l'énergie des ultrasons transmise par l'eau engendre, sur le cil maquillé, des cavitations qui ont pour effet de le nettoyer. L'intérêt de cette technique, par rapport à l'action d'un courant d'eau sur le cil, réside dans sa rapidité.

Le mode opératoire général est le suivant 5 cils humains d'une meme personne sont fixés par leur racine à une plaque de carton. Les cils sont maquillés manuellement avec un mascara et ils sont mis à sécher pendant 15 minutes. Ensuite, ils sont immergés dans une cuve remplie d'eau et soumis aux ultrasons pendant 5 minutes, puis pendant 7 autres minutes. Des photographies sont prises avant le maquillage, juste avant l'immersion et au bout des 10 minutes d'immersion. Un agrandissement des clichés suivi d'une étude planimétrique permet, par comparaison, de connaître la perte du mascara au bout de 10 minutes.

On soumet à ce test les mascaras A et B tels que définis à l'exemple 10, et un mascara C (selon l'invention), dans lequel la solution d'hydrolysat de kératine à 13 % en poids de matières sèches du mascara B est remplacée par une solution aqueuse à 3 % en poids de matières sèches d'un copolymère (hydroxyéthylcellulose quaternisée/polymère acrylique) (rapport pondéral 1/5). On mesure le pourcentage P de perte de mascara sur des cils immergés dans une cuve à ultrasons pendant 10 minutes suivant la procédure expérimentale ci-dessus définie. Les résultats sont consignés dans le tableau ci-après :

| Mascara | P |
|---------|---|
| A | 45 |
| B | 23 |
| C | 18 |

Les pourcentages de perte au bout de 10 minutes montrent de très bonnes caractéristiques de résistance à l'eau pour les mascaras B et C selon l'invention. On suppose que les polymères hydrosolubles et filmogènes incorporés, conformément à l'invention, plastifient la structure du mascara et augmentent sa rémanence à l'eau.

Exemple 13 : (Comparatif)

La comparaison des photographies en microscopie électronique de cils enrobés des mascaras A et B, tels que définis à l'exemple 10, montre, avec le mascara B selon l'invention, un gainage du cil après maquillage plus homogène et plus régulier qu'avec le mascara A.

EXEMPLE 14 : (Comparatif)

On a préparé deux mascaras D et E contenant comme polymère filmogène du pyrrolidone carboxylate de chitosonium vendu sous la dénomination "KYTAMER PC" par la société "AMERCHOL" dans le mascara D, le polymère étant introduit sous forme de solution dans l'eau et le mascara E sous forme anhydre.

Ces deux mascaras ont la composition suivante :

| Constituants | D | E |
|---|---|---|
| Cire de paraffine | 22,0 | 24,20 |
| Acide stéarique | 3,0 | 3,30 |
| Amidon | 1,0 | 1,1 |
| Oxyde de fer | 5,0 | 5,5 |
| Isoparaffine | 48,50 | 53,50 |
| Montmorillonite | 10,0 | 10,9 |
| Pyrrolidone carboxylate de chitosonium vendu sous la dénomination "KYTAMER PC" par la société "AMERCHOL" | 1,50 | 1,50 |
| Eau | 9,00 | - |

La comparaison de photographies au microscope d'une couche de mascara D et de mascara E montre que le mascara D est une pate souple homogène, tandis que le mascara E est hétérogène et présente des grains durs de polymère filmogène non solubilisé.

## Revendications

1. Composition résistante à l'eau, pour le revêtement des cils, contenant au moins une cire, au moins un agent de consistance, et au moins un solvant organique volatil, caractérisée par le fait qu'elle contient, en outre, de 1 à 35 % par rapport au poids total de la composition d'une solution aqueuse d'au moins un polymère filmogène hydrosoluble, la concentration en polymère(s) filmogène(s) hydrosoluble(s) dans la solution étant comprise entre 0,1 et 55 % en poids de matière active et qu'elle ne contient pas d'agent émulsionnant.

2. Composition selon la revendication 1, caractérisée par le fait que le (ou les) polymère(s) filmogène(s) est (ou sont) choisi(s) dans le groupe formé par les dérivés de kératine ; les dérivés de chitine ou de chitosane, anioniques, cationiques, amphotères ou non-ioniques ; les dérivés de cellulose; les polymères et copolymères acryliques ; les polyvinylpyrrolidones et les copolymères vinyliques ; les polymères naturels ; les polymères de l'éthylène ; et les silicones oxyéthylénées.

3. Composition selon l'une des revendications 1 ou 2, caractérisée par le fait que la (ou les) cire(s) est (sont) choisie(s) dans le groupe formé par les cires animales, végétales, minérales, synthétiques, et les fractions diverses de cires naturelles, toutes ces cires ayant un point de fusion compris entre 60 et 110°C et une pénétration à l'aiguille, à 25°C, comprise entre 3 et 40, telle que mesurée selon les normes ASTM D5 ou NFT 004.

4. Composition selon la revendication 3, caractérisée par le fait que la (ou les) cire(s) est (ou sont) solide(s) et rigide(s) à une température inférieure à 50°C.

5. Composition selon l'une des revendications 1 à 4, caractérisée par le fait que la concentration en cire(s) par rapport au poids total de la composition est comprise entre 2 et 40 % en poids.

6. Composition selon l'une des revendications 1 à 5, caractérisée par le fait que l'(les) agent(s) de consistance est (sont) choisi(s) dans le groupe formé par les argiles modifiées organiquement.

7. Composition selon l'une des revendications 1 à 6, caractérisée par le fait que la concentration en agent(s) de consistance par rapport au poids total de la composition est comprise entre 5 et 15 % en poids.

8. Composition selon l'une des revendications 1 à 7, caractérisée par le fait que le (ou les) solvant(s) organique(s) volatil(s) est (ou sont) choisi(s) dans le groupe formé par l'isoparaffine, l'essence de térébenthine, l'alcool isopropylique, l'alcool éthylique, le white spirit et les dérivés de silicone volatils.

9. Composition selon l'une des revendications 1 à 8, caractérisée par le fait que la concentration en solvant(s) organique(s) volatil(s) par rapport au poids total de la composition est comprise entre 35 et 50 % en poids.

10. Composition selon l'une des revendications 1 à 9, caractérisée par le fait qu'elle contient jusqu'à 10 % en poids, par rapport au poids total de la composition, d'au moins une charge pulvérulente choisie dans le groupe formé par le talc, l'amidon, le kaolin et les polyamides.

11. Composition selon l'une des revendications 1 à 10, caractérisée par le fait qu'elle contient jusqu'à 20 % en poids, par rapport au poids total de la composition, d'au moins un pigment choisi dans le groupe formé par les pigments minéraux, les pigments organiques, les pigments nacrés et les pigments enrobés.

12. Composition selon l'une des revendications 1 à 11, caractérisée par le fait qu'elle contient au moins un additif classique dans les compositions de maquillage pour les cils, choisi dans le groupe formé par les adoucissants, les conservateurs, les séquestrants, les parfums, les épaississants, les huiles, les silicones, les agents de cohésion, les polymères non-filmogènes, les agents alcalinisants ou acidifiants, les vitamines et les acides aminés.

13. Procédé de préparation d'une composition selon l'une des revendications 1 à 12, caractérisé par le fait que :
    - dans une première étape, on mélange les composants de la phase grasse et les éventuels additifs liposolubles ;
    - dans une seconde étape, on ajoute au mélange ainsi obtenu, les charges et/ou pigments éventuels, puis le (ou les) solvant(s) organique(s) volatil(s) ; et
    - dans une troisième étape, on disperse dans le mélange résultant la phase aqueuse contenant le (ou les) polymère(s) filmogène(s) hydrosoluble(s) et les éventuels additifs et/ou ingrédients actifs hydrosolubles.

**Patentansprüche**

1. Wasserbeständige Zusammensetzung zum Auftragen auf die Wimpern, enthaltend wenigstens ein Wachs, wenigstens ein Konsistenzmittel und wenigstens ein flüchtiges organisches Lösungsmittel, **dadurch gekennzeichnet,** daß
es zusätzlich 1 bis 35 %, bezogen auf das Gesamtgewicht der Zusammensetzung, einer wäßrigen Lösung wenigstens eines filmbildenden, wasserlöslichen Polymers enthält, wobei die Konzentration des (der) filmbildenden, wasserlöslichen Polymers (Polymere) in der Lösung im Bereich von 0,1 bis 55 Gew.-% aktives Material liegt und daß es kein Emulgierungsmittel enthält.

2. Zusammensetzung nach Anspruch 1,
dadurch gekennzeichnet, daß das (die) filmbildende(n) Polymer(e) ausgewählt ist (sind) unter Keratinderivaten; anionischen, kationischen, amphoteren oder nicht-ionischen Derivaten von Chitin oder Chitosan; Cellulosederivaten; Acrylpolymeren und -copolymeren; Polyvinylpyrrolidonen und Vinylcopolymeren; natürlichen Polymeren; Ethylenpolymeren; und oxyethylenierten Silikonen.

3. Zusammensetzung nach einem der Ansprüche 1 oder 2, dadurch gekennzeichnet, daß das (oder die) Wachs(e) ausgewählt ist (sind) unter tierischen, pflanzlichen, mineralischen oder synthetischen Wachsen, und verschiedenen Fraktionen natürlicher Wachse, wobei die Wachse einen Schmelzpunkt im Bereich von 60 bis 110°C und bei 25°C eine Nadel-Penetration im Bereich von 3 bis 40, bestimmt in Übereinstimmung mit den Normen ASTM D5 oder NFT 004, aufweisen.

4. Zusammensetzung nach Anspruch 3, dadurch gekennzeichnet, daß das (oder die) Wachs(e) bei einer Temperatur von weniger als 50°C fest und hart ist (oder sind).

5. Zusammensetzung nach einem der Ansprüche 1 bis 4, dadurch gekennzeichnet, daß die Wachskonzentration, bezogen auf das Gesamtgewicht der Zusammensetzung, 2 bis 40 Gew.-% beträgt.

6. Zusammensetzung nach einem der Ansprüche 1 bis 5, dadurch gekennzeichnet, daß das (die) Konsistenzmittel ausgewählt ist (sind) unter organisch modifizierten Tonmineralien.

7. Zusammensetzung nach einem der Ansprüche 1 bis 6, dadurch gekennnzeichnet, daß die Konzentration der (des) Konsistenzmittel(s), bezogen auf das Gesamtgewicht der Zusammensetzung, 5 bis 15 Gew.-% beträgt.

8. Zusammensetzung nach einem der Ansprüche 1 bis 7, dadurch gekennzeichnet, daß das (oder die) flüchtige(n) organische(n) Lösungsmittel ausgewählt ist (oder sind) unter Isoparaffin, Terpentinöl, Isopropylalkohol, Ethylalkohol, White Spirit und flüchtigen Silikonderivaten.

9. Zusammensetzung nach einem der Ansprüche 1 bis 8, dadurch gekennzeichnet, daß die Konzentration der (des) flüchtigen organischen Lösungsmittel(s), bezogen auf das Gesamtgewicht der Zusammensetzung, 35 bis 50 Gew.-% beträgt.

10. Zusammensetzung nach einem der Ansprüche 1 bis 9, dadurch gekennzeichnet, daß sie bis zu 10 Gew.-%, bezogen auf das Gesamtgewicht der Zusammensetzung, wenigstens eines pulverförmigen Füllstoffs enthält, ausgewählt unter Talkum, Stärke, Kaolin und Polyamiden.

11. Zusammensetzung nach einem der Ansprüche 1 bis 10, dadurch gekennzeichnet, daß sie bis zu 20 Gew.-%, bezogen auf das Gesamtgewicht der Zusammensetzung, wenigstens eines Pigments enthält, ausgewählt unter mineralischen Pigmenten, organischen Pigmenten, perlmuttartigen Pigmenten und beschichteten Pigmenten.

12. Zusammensetzung nach einem der Ansprüche 1 bis 11, dadurch gekennzeichnet, daß sie wenigstens einen Zusatz enthält, der üblicherweise in Zusammensetzung für Wimpernschminke enthalten ist und ausgewählt ist unter weichmachenden Mitteln, Konservierungsmitteln, Sequestriermitteln, Parfums, Verdickungsmitteln, Ölen, Silikonen, Kohäsionsmitteln, nichtfilmbildenden Polymeren, alkalinisierende oder acidifizierenden Mitteln, Vitaminen und Aminosäuren.

13. Verfahren zur Herstellung einer Zusammensetzung nach einem der Ansprüche 1 bis 12, dadurch gekennzeichnet, daß man:
    - in einem ersten Schritt die Bestandteile der Fettphase und gegebenenfalls die fettlöslichen Additive miteinander vermischt;
    - in einem zweiten Schritt zu der so erhaltenen Mischung die Füllstoffe und/oder gegebenenfalls die Pigmente und anschließend das (oder die) organische(n), flüchtige(n) Lösungsmittel hinzugibt; und
    - in einem dritten Schritt in der resultierenden Mischung die wäßrige Phase dispergiert, welche das (oder die) filmbildende(n) wasserlösliche(n) Polymer(e) und gegebenenfalls die wasserlöslichen Zusätze und/oder aktiven Bestandteile enthält.

## Claims

1. Water-resistant composition, for covering the eyelashes, containing at least one wax, at least one consistency agent, and at least one volatile organic solvent, characterized by the fact that it contains, in addition, 1 to 35%, relative to the total weight of the composition, of an aqueous solution of at least one water-soluble film-forming polymer, the concentration of water-soluble film-forming polymer(s) in the solution being between 0.1 and 55% by weight of active material and that it does not contain an emulsifying agent.

2. Composition according to Claim 1, characterized by the fact that the film-forming polymer(s) is (are) chosen from the group consisting of keratin derivatives; anionic, cationic, amphoteric or non-ionic keratin or chitosan derivatives; cellulose derivatives; acrylic polymers and copolymers; polyvinylpyrrolidones and vinyl copolymers; natural polymers; ethylene polymers; and oxyethylenated silicones.

3. Composition according to one of Claims 1 or 2, characterized in by the fact that the wax(es) is (are) chosen from the group consisting of animal, vegetable, mineral or synthetic waxes, and the various fractions of natural waxes, all these waxes having a melting point of between 60 and 110°C and a needle penetration, at 25°C, of between 3 and 40, as measured according to the ASTM D5 or NFT 004 standards.

4. Composition according to Claim 3, characterized by the fact that the wax(es) is (are) solid and rigid at a temperature of less than 50°C.

5. Composition according to one of Claims 1 to 4, characterized by the fact that the concentration of wax(es) relative to the total weight of the composition is between 2 and 40% by weight.

6. Composition according to one of Claims 1 to 5, characterized by the fact that the consistency agent(s) is (are) chosen from the group consisting of organically modified clays.

7. Composition according to one of Claims 1 to 6, characterized by the fact that the concentration of consistency agent(s) relative to the total weight of the composition is between 5 and 15 % by weight.

8. Composition according to one of Claims 1 to 7, characterized by the fact that the volatile organic solvent(s) is (are) chosen from the group consisting of isoparaffin, turpentine oil, isopropyl alcohol, ethyl alcohol, white spirit and volatile silicone derivatives.

9. Composition according to one of Claims 1 to 8, characterized by the fact that the concentration of volatile organic solvent(s) relative to the total weight of the composition is between 35 and 50% by weight.

10. Composition according to one of Claims 1 to 9, characterized by the fact that it contains up to 10% by weight, relative to the total weight of the composition, of at least one pulverulent filler chosen from the group consisting of talc, starch, kaolin and polyamides.

11. Composition according to one of Claims 1 to 10, characterized by the fact that it contains up to 20% by weight, relative to the total weight of the composition, of at least one pigment chosen from the group consisting of inorganic pigments, organic pigments, pearlescent pigments and coated pigments.

12. Composition according to one of Claims 1 to 11, characterized by the fact that it contains at least one additive, conventional in make-up compositions for the eyelashes, chosen from the group consisting of emollients, preservatives, sequestrants, perfumes, thickeners, oils, silicones, cohesion agents, non-film forming polymers, alkalinizing or acidifying agents, vitamins and amino acids.

13. Process for preparing a composition according to Claims 1 to 12, characterized by the fact that:
    - in a first stage, the components of the fatty phase and the optional fat-soluble additives are mixed;
    - in a second stage, the optional fillers and/or pigments, then the volatile organic solvent(s) are added to the mixture thus obtained; and
    - in a third stage, the aqueous phase containing the water-soluble film-forming polymer(s) and the optional water-soluble additives and/or active ingredients are dispersed in the resulting mixture.